(19) Europäisches Patentamt
European Patent Office
Office européen des brevets

(11) Publication number: 0 211 033 B1

(12) **EUROPEAN PATENT SPECIFICATION**

(45) Date of publication of patent specification: **17.07.91 Bulletin 91/29**

(51) Int. Cl.$^5$: **C12N 9/06**

(21) Application number: **86900870.6**

(22) Date of filing: **10.01.86**

(86) International application number: **PCT/SE86/00006**

(87) International publication number: **WO 86/04087 17.07.86 Gazette 86/17**

# (54) A D-AMINO ACID OXIDASE AND METHOD FOR ISOLATION THEREOF.

(30) Priority: **11.01.85 SE 8500157**

(43) Date of publication of application: **25.02.87 Bulletin 87/09**

(45) Publication of the grant of the patent: **17.07.91 Bulletin 91/29**

(84) Designated Contracting States: **AT BE CH DE FR GB IT LI LU NL SE**

(56) References cited:
**US-A- 3 658 649**
**US-A- 3 801 458**
**Chemical abstracts, Vol 102, 1985, abstract No 127 712p, Szweicer et al. Biotechnol. Lett. 1985 7(1), 1-7 (Eng.)**
**Chemical abstracts, Vol 103, 1985, abstract No 100 771r Kubicek-Pranz et al. J. Appl. Biochem. 1985, 7(2), 104-13 (Eng.)**
**Chemical abstracts, Vol 103, 1985, abstract No 103 412k, Kubicek-Pranz et al., Can. J. Microbiol. 1985, 31(7), 625-8 (Eng.)**
**Analytical Biochemistry, Vol. 71, pp. 214-222, 1976**

(73) Proprietor: **MOSBACH, Klaus, Prof. Dr.**
**Rebbergstrasse 86**
**CH-8102 Oberengstringen (CH)**

(72) Inventor: **SZWAJCER, Estera**
**Tillämpad Biokemi Kemicentrum**
**Box 740 S-220 07 Lund (SE)**
Inventor: **MOSBACH, Klaus**
**Rebbergstrasse 83**
**CH-8102 Oberengstringen (CH)**

(74) Representative: **Wiklund, Ingrid Helena et al**
**AWAPATENT AB P.O. Box 5117**
**S-200 71 Malmö (SE)**

EP 0 211 033 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a D-amino acid oxidase which is active against cephalosporin C, and a method for isolating it from Trigonopsis variabilis.

There is a long-standing considerable interest in using amino acid oxidase activity for forming α-keto acids from their corresponding amino acids since these are of interest as a nutritional supplement for patients suffering from kidney insufficiency. Both D-amino acid oxidase activity found in the yeast Trigonopsis variabilis (Brodelius, P., Nilsson, K., and Mosbach, K., 1981, Appl. Biochem, Biotechnol. 6, 293-308) and L-amino acid oxidase activity found in the bacterium Providencia (Szwajcer, E., Brodelius, P., and Mosbach, K. (1982). Enzyme Microb. Technol. 4, 409-413) have been used. D-amino acid oxidase also exhibited activity against cephalosporin C, by oxidatively deaminating the latter to keto adipic 7-aminocephalosporanic acid. The same activity was also reported in US-PS-3,658,649. However, the extract used was obtained only by ammonium sulfate precipitation and thus represents crude preparation comprising several amino acid oxidases. A number of different organisms have been examined in respect of the latter activity, including E. coli, Pseudomonas species, Aerobacter species, Candida tropicalis, Penicillium rocforti, Aspergillus flavus and A. niger, Neurospora crassa, Nocardia, Citrobacter and Trigonopsis variabilis. Only Trigonopsis variabilis and Citrobacter could deaminate cephalosporin C to keto adipic 7-aminocephalosporanic acid. The activity found in Citrobacter was very low and appeared to be membrane-bound, while the enzyme from Trigonopsis was present in the cytoplasm and at a much higher level. It should be mentioned in this context that the activity against cephalosporin C was also found with the enzyme obtained from hog kidney (Mazzeo, P., and Romeo, A. (1972). J.C.S. Perkin I(P3), 2532). 7-Aminocephalosporanic acid has great industrial interest as basic moiety for the preparation of semisynthetic cephalosporins by analogy with 6-aminopenicillanoic acid for the preparation of semisynthetic penicillins. An acylase has been reported in the literature (Shibyva, Y., Matsumoto, K., and Fuji, T. (1981). Agr. Biol. Chem. 45, (1561-1567) which hydrolyzes the side-chain of glutaryl-7-aminocephalosporanic acid. The latter compound is spontaneously formed from keto adipic-7-aminocephalosporanic acid by the hydrogen peroxide simultaneously formed. The following diagram illustrates the formation of 7-aminocephalosporanic acid, under the influence of two enzymes :

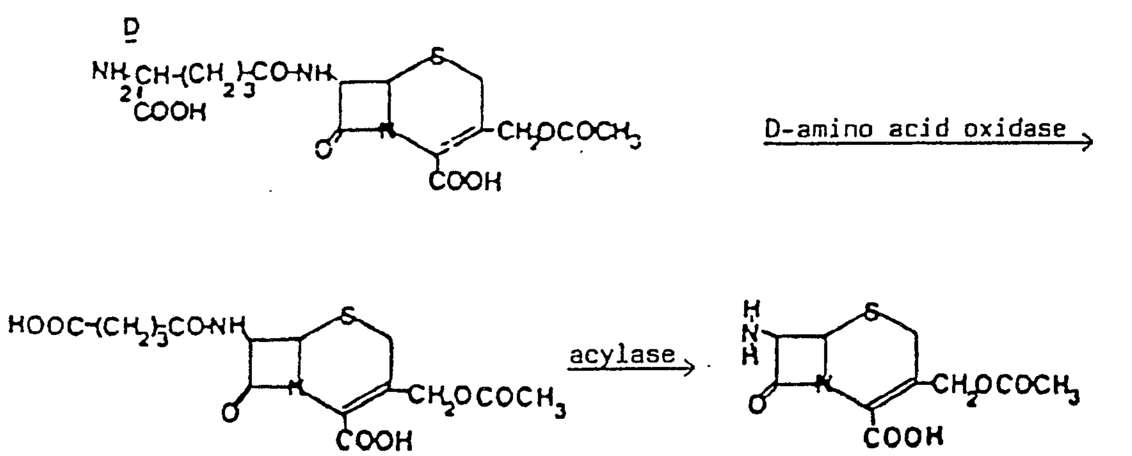


glutaryl-7-aminocephalosporanic acid

7-aminocephalosporanic acid

The present invention has for its object to produce a Trigonopsis variabilis D-amino acid oxidase in pure form, as seen by electrophoresis, said D-amino acid oxidase having specific activity against cephalosporin C characterized in that said D-amino acid oxidase has an isoelectric point of about 4.6 and exist in its active form as a dimer with a molecular weight of about 86,000 and with two subunits.

Another object of the invention is to provide a method for isolating a D-amino acid oxidase from Trigonopsis variabilis.

The simple method for purifying D-amino acid oxidase to homogeneity according to the invention is carried out in three steps. Thus, the method is characterized in that it comprises

(a) acidifying and heating a crude cell extract of Trigonopsis variabilis to obtain a precipitate and supernatant fraction ;

(b) treating said supernatant fraction obtained in step (a) with sufficient ammonium sulfate to obtain a second precipitate, said second precipitate containing the D-amino acid oxidase of claim 1 ; and

(c) resuspending the precipitate obtained in step (b) and collecting the D-amino acid oxidase by isoelectric precipitation at a pH of about 4.6.

The acidification according to step (a) above can be performed by adding acetic acid to the crude cell extract. The cell extract is acidified to a pH of about 4 to 6, preferably about 5.1 to 5.3 and most preferably about 5.3.

In a further embodiment, any precipitate formed after acidification is removed prior to heating. Further, D,L-methionine can be added prior to heating, preferably in an amount to obtain a final concentration of about 25 mM.

The crude extract is heated in step (a) above to a temperature of about 40 to about 60°C, preferably about 40 to about 50°C and most preferably to about 50°C.

In another preferred embodiment, the supernatant fraction of step (a) is dialyzed prior to the treatment with ammonium sulfate, preferably against a buffer comprising 20 mM sodium pyrophosphate, pH 8.3.

In still another preferred embodiment, the supernatant fraction of step (a) is concentrated after dialysis prior to the treatment with ammonium sulfate. This concentration is preferably accomplished by evaporation.

The treatment with ammonium sulfate may in a preferred embodiment comprise

(i) adding ammonium sulfate to said supernatant fraction to obtain an ammonium sulfate concentration of about 30% by weight and removing the resulting precipitate ; and

(ii) adding additional ammonium sulfate to the supernatant of (a) to obtain an ammonium sulfate concentration of about 55% and collecting the resulting precipitate.

The second D-amino acid oxidase-containing precipitate in step (c) above can be dialyzed prior to collecting by isoelectric precipitation, preferably against a buffer comprising 20 mM sodium pyrophosphate, pH 8.3.

The isoelectric precipitation comprises :

(a) dialyzing said second precipitate against a buffer that comprises 25 mM sodium acetate, pH 5.1, and removing any precipitate that remains after said dialyzing step ; and

(b) dialyzing the solution obtained from said dialyzing step against a buffer that comprises 100 mM sodium acetate, pH 4.6, and collecting the purified D-amino acid oxidase precipitate.

The D-amino acid oxidase obtained by the isoelectric precipitation may be further purified by gel electrophoresis.

The invention also comprises an immobilized form of the D-amino acid oxidase, preferably conjugated with cyanogen-bromide-activated Sepharose.

In a further embodiment, the method of the invention is characterized in that

(a) cells of Trigonopsis variabilis are disintegrated for forming a crude extract which is acidified ;

(b) the acidified crude extract is heated to 50°C, and the precipitate formed is removed and the supernatant dialyzed ;

(c) the product from (b) is precipitated with ammonium sulfate and dialyzed ; and

(d) D-amino acid oxidase is isolated by isoelectric precipitation.

In a still further embodiment, the method of the invention comprises :

(a) acidifying a crude cell extract of Trigonopsis variabilis by adding acetic until the pH of the extract is about 5.3 to form a precipitate and supernatant fraction ;

(b) adding D,L-methionine to said supernatant to a final concentration of 25 mM ;

(c) heating said supernatant to 50°C and maintaining it at 50°C for ten minutes to form a second precipitate and supernatant fraction ;

(d) dialyzing said supernatant obtained in step (c) against 20 mM sodium pyrophosphate buffer, pH 8.3 ;

(e) adding sodium ammonium sulfate to the solution prepared in step (d) to a final concentration of 30% and removing the resulting precipitate ;

(f) adding solid ammonium sulfate to the solution prepared in step (e) to a final concentration of 55% and collecting the resulting precipitate ;

(g) dialyzing said precipitate prepared in step (f) first against 20 mM sodium pyrophosphate buffer, pH 8.3, then against 25 mM sodium acetate buffer, pH 5.1, and removing any undissolved precipitate that remains after dialysis ; and

(h) dialyzing the solution obtained in step (g) against 100 mM sodium acetate buffer, pH 4.6, and collecting the D-amino acid oxidase containing precipitate.

The invention will now be described in greater detail in an Example, with reference to the accompanying drawings.

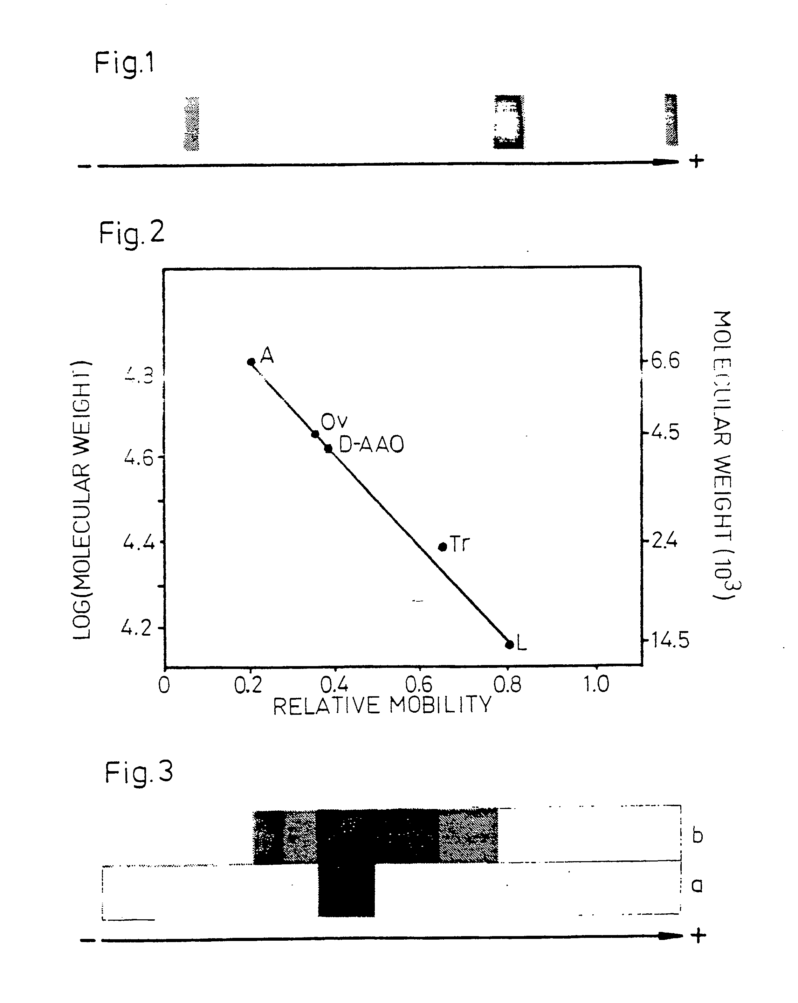

Fig. 1 shows polyacrylamide gel electrophoresis of purified D-amino acid oxidase. Gel electrophoresis of 25 μg of pure protein in sodium dodecyl sulphate shows only one protein band (the dark minor band seen to the left indicates the borderline of the two "fused" gels). The marker added to the gels was cut out prior to protein staining.

Fig. 2 shows the result of the determination of the molecular weight of D-amino acid oxydase from Trigonopsis variabilis. SDS gel electrophoresis was carried out in 12% polyacrylamide according to Laemmli, (Laemmli, U.K. (1970). Nature 227, 680-685). The following protein markers were used : A = albumin (66,000) OV = ovalalbumin (45,000), Tr = trypsinogen (24,000), and L = lysozyme (14,500).

Fig. 3 shows activity staining of the gels in respect of D-amino acid oxidase. The illustrated fraction is taken from Step 3 according to the invention. The gels were incubated : a = with cephalosporin C, b = with D-leucine. The analysis was made as described below.

Materials and methods

Materials :

Peroxidase (Type II, from horse-radish), all amino acids, cephalosporin C, dinitrophenyl hydrazine and o-dianisidine were obtained from Sigma (St. Louis, Mo, USA). Acrylamide and N,N'-methylene-bis-acrylamide were obtained from Merck-Schuchardt (Munich, Germany). N,N,N',N'-tetramethylethylenediamine, ammonium persulphate and sheets precoated with 0.25 mm of silica gel $F_{254}^{R}$ were obtained from Merck (Darmstadt, Germany). Growth media were products from Difco (Detroit, USA). The oxygen electrode was obtained from Rank Brothers Bottisham (Cambridge, Great Britain).

Methods

Analytical isoelectric focusing in polyacrylamide gel was carried out, using the LKB 1804-101$^{R}$ system. The carrier ampholytes used had a pH range of 3.5-9.5. Isoelectric focusing was performed in accordance with a leaflet from LKB regarding ampholine PAG plates for analytical electrofocusing on polyacrylamide gels (LKB-Produkter AB, Bromma, Sweden, 1979).

Iron was determined at the Department of Analytical Chemistry, University of Lund, by atomic absorption spectrophotometry.

Culture conditions :

The microorganism used in the studies was the yeast Trigonopsis variabilis, the same as used by Brodelius et al. (Brodelius, P., Nilsson, K., and Mosbach, K. (1981). Appl. Biochem. Biotechnol. 6, 293-308). Larger batches of cells were obtained from 8-litre cultures in a fermentor. The growth medium was composed of yeast extract (1%), malt extract (1.5%) supplemented with 0.2% DL-methionine, pH 6.0, and incubated for 44 hours at 28°C. The stock culture was maintained on slants made up of the same medium containing 2% agar. The cells were collected by centrifugation at 4000 g for 30 min., washed and stored in the frozen state until used. The average yield of the cells from different fermentations was in the range of 45-50 g cell paste.

Streptomyces and Nocardia strains were obtained from the Departement of Microbiology, Institute of Immunology, Wroclaw, Poland. Bacteria and fungi were obtained from ATCC.

Electrophoresis

For analytical purposes, disc polyacrylamide gel electrophoresis was carried out according to Hedrick and Smith (Hedrick, J.L., and Smith, A.J. (1968). Arch. Biochem. Biophys. 126, 155-164) and according to Laemmli (Laemmli, U.K. (1970). Nature 227, 680-685) for SDS-containing gels. Electrophoresis was performed at 8-10°C. Initially, a low current of 2 mA per tube was supplied until the dye had migrated into the separation gel, whereupon the current was increased to a constant value of 4 mA per tube. The diameter and length of the tube was 0.8 cm and 9.5 cm, respectively. For locating the protein, the gels were stained with Coomassie Brilliant Blue R.

Enzymatic activity on the gels was located according to Hedrick and Smith (see above), the gels being stained by detection of the formed hydrogen peroxide, using o-dianisidine. The gels were incubated in 0.1 M sodium phosphate buffer, pH 7.2, containing 5 mM cephalosporin C (or other amino acids), peroxidase (0.025%), and o-dianisidine (0.025%). Incubation wass carried out for 30-60 min. until the reddish crown dye had been formed.

For preparative enzyme isolation, a special equipment has been designed by the inventors. The diameter of the glass tube was 2 cm and the length of the tube was 14 cm. The gel (8% polyacrylamide) was prepared according to Hedrick and Smith (see above), using 0.19 M Tris-glycine buffer, pH 8.3. The upper gel was polymerized in the presence of ammonium persulphate and TEMED instead of riboflavin (the amount of catalysts was taken according to Laemmli (see above)). Electrophoresis was carried out for 3-4 hours at 10°C, using 20 mA during the first 30 min., followed by 40 mA.

Molecular weight determination :

The molecular weight of the protein was determined as described by Hedrick and Smith (see above) and SDS gel electrophoresis was performed according to Laemmli (see above). For the first method, use was made of molecular weight markers for non-denaturated polyacrylamide gel electrophoresis (SIGMA) and, for the latter, use was made of SDS molecular weight markers. Protein determination was made according to Lowry et al. (Lowry, O.H., Rosebrough, N.J., Ferr, A.L., and Randall, R.J. (1951). Biol. Chem. 193, 265-275).

Analysis in respect of D-amino acid oxidase activity :

By determining the rate of oxygen consumption with a Rank oxygen electrode, D-amino acid oxidase was assayed at 50°C for 5-10 min., during which time no enzyme denaturation took place. The assay mixture contained in a final volume of 2 ml, 1.8 ml (20 mM) sodium pyrophosphate, pH 8.0, 0.1 ml (100 mM) cephalosporin C and the appropriate amount of enzyme to provide the correct initial rate of oxygen consumption. 1 unit (U) corresponds to the uptake of 1 µmole of oxygen/min. under the conditions used. From time to time, the activity was also checked by measuring the amount of formed keto acid with a colorimetrical method, at 30°C, using 2,4-dinitrophenyl hydrazine as well as with o-dianisidine-peroxidase (see above).

EXAMPLE

Purification of D-amino acid oxidase from Trigonopsis variabilis :

All operations were performed at 5°C.

Step 1 :

Preparation of crude extract :

32 g of frozen cell paste (−20°C) was thawed and suspended in 1.5 volumes of 20 mM sodium pyrophosphate buffer, pH 8.3. The cell suspension was mixed with an equal volume of powdered dry ice and disintegrated in a mixer. D-amino acid oxidase was released together with other soluble proteins. The disrupted cells were centrifuged for 30 min. at 12,000 g. The disrupted cells were washed several times with a new aliquot of buffer and centrifuged. The collected supernatants were acidified to pH 5.3 with 2 m acetic acid, and the resulting precipitate was removed by centrifugation for 30 min. at 12,000 g.

Step 2 :

Heat precipitation :

To the acidic supernatant (730 ml) from Step 1 was added 25 mM of DL-methionine to protect the enzyme. The supernatant was heated to 50°C and maintained at this temperature for 10 min. in a water bath. The resulting precipitate was spun down for 30 min. at 12,000 g and the precipitate was discarded. The supernatant was dialyzed against 20 mM sodium pyrophosphate buffer, pH 8.3, overnight.

Step 3 :

Ammonium sulphate precipitation :

The sample from Step 2 was concentrated by blowing warm air over a dialysis bag (containing the sample) to increase the amount of protein per ml (1%). The pH of the sample was lowered with 2 M acetic acid to pH 6.3 and thereafter mixed for 1-2 hours with solid ammonium sulphate. The fraction salted out with 30% ammonium sulphate was left to form a precipitate and thereafter centrifuged for 30 min. at 12,000 g. The precipitate was discarded and the supernatant (after adjusting to pH 6.0) was mixed with solid ammonium sulphate (55%). The precipitate formed after 2 hours was centrifuged for 30 min. at 12,000 g and dialyzed against 20 mM sodium pyrophosphate, pH 8.3.

Step 4 :

Isoelectric precipitation :

The sample from Step 3 (10.3 ml) was dialyzed against 25 mM sodium acetate buffer, pH 5.1, for 12 hours. the resulting precipitate was removed by centrifugation for 30 min. at 12,000 g. The precipitate contained traces of enzymatic activity. The supernatant was precipitated once more by dialysis against 0.1 M sodium acetate buffer, pH 4.6, for 12 hours. The precipitate formed contained most of the D-amino acid oxidase activity. The precipitate was dissolved in 20 mM pyrophosphate buffer, pH 8.3, and dialyzed against the same buffer overnight.

Preparative disc gel electrophoresis :

The sample from Step 4 was used for the preparative gel electrophoresis. 5 mg of the protein from Step 4 was used for the electrophoretic test. The band showing amino acid oxidase activity against cephalosporin C was cut out and homogenized for 5 min. with a Potter homogenizer (1000 rev./min.) in 20 mM pyrophosphate buffer, pH 8.3, whereupon the suspension obtained was centrifuged at 1000 g for 20 min. The gel was washed 3 times with an equal amount of buffer and the collected supernatants were concentrated by means of warm air, as above, to a final volume of 3 ml and used for further studies.

RESULTS AND DICUSSION

TABLE 1

Purification of D-amino acid oxidase from Trigonopsis variabilis. The activity was measured against cephalosporin C. The protein content was determined as described by Lowry et al. (see above).

| Treatment steps | Total activity | Protein | Specific activity | Purification | Yield |
|---|---|---|---|---|---|
| | Units | mg/ml | U/mg | Fold | % |
| 1. Crude extract | 384 | 3.5 | 0.15 | 1 | 100 |
| 2. Heat precipitation | 308 | 5.6 | 1.0 | 6.5 | 80 |
| 3. $(NH_4)_2SO_4$ precipitation (30-55%) | 300 | 6.0 | 4.9 | 33.0 | 78 |
| 4. Isoelectric precipitation, pH 4.6 | 154 | 2.8 | 5.8 | 39 | 40 |

The present invention provides a simple method for purifying D-amino acid oxidase to homogeneity. Preparative gel electrophoresis after the isoelectric precipitation step removed two additional weak bands observed on gel electrophoresis. The specific activity decreased from 5.8 U/mg (Table 1) to 3.8 U/mg. It can be assumed that this is due to partial denaturation occurring during extracticn or to loss of cofactors not yet identified.

Polyacrylamide gel electrophoresis in sodium dodecyl sulphate yielded a single band (Fig. 1) of a molecular weight of about 43,000 (Fig. 2). The molecular weight of the native protein, determined according to Hedrick and Smith (Hedrick, J.L., and Smith, A.J, (1968). Arch. Biochem. Biophys. 126, 155-164), was estimated at about 86,000.

The D-amino acid oxidase thus exists in its active form as a dimer with a molecular weight of about 86,000 and with two subunits.

Enzymatic staining of the gels after incubation with D-leucine and cephalosporin C, respectively, showed only one band with the sample obtained after preparative gel electrophoresis, whereas the less purified preparation (Step 3) yielded three major bands after incubation with D-leucine and only one band with cephalosporin C (Fig. 3). This indicates the presence of several D-amino acid oxidases or isoenzymes in Trigonopsis, but only one of them exhibited activity against cephalosporin C. There is one report in the literature on the purification of D-amino acid oxidase from Trigonopsis variabilis (Berg, C.P., and Rodden, F.A. (1976) Anal. Biochem. 71, 214-222). However, the authors tested this protein only for activity against D-leucine. They did not check the homogeniety of the enzyme preparation. The procedure yields an impure mixture, some of the proteins of which are likely to represent amino acid oxidases.

The isolated D-amino acid oxidase having activity against cephalosporin C exhibited the following properties : The isoelectric joint of the enzyme was about 4.6. Atomic absorption analysis strongly indicates that the enzyme contains two moles of iron which corresponds to one mole per subunit. After dialysis against 10 mM EDTA and 1 mM o-phenanthroline, respectively, the activity remained unchanged. Arsenite treatment reduced the activity to 46% and cyanide to 34% at a concentration of 0.5 mM. The $K_m$ values for cephalosporin C, phenylalanine, alanine, methionine and leucine were 13, 10, 76, 0.76 and 0.12, respectively. The sample obtained after preparative gel electrophoresis at a concetration of 5 mg/ml did not exhibit a spectrum similar to that of flavin-dependent enzymes, as may have been expected, since the amino acid oxidase obtained from hog kidney which is active against cephalosporin C is FAD-dependent (Mazzeo, P., and Romeo, A. (1972). J.C.S. Perkin. I(P3), 2532). The addition of various flavin cofactors, such as FMN or FAD, did not increase the reduced specific activity. Attempts to isolate flavins from the enzyme were unsuccessful. Thus, extensive dialysis against KBr in acid solution (Mayhew, S.G. (1971), Biochim. Biophys. Acta 235, 289-302), at alkaline pH (Massey, V., and Curti, B. (1966). J. Biol.Chem. 241, 3417-3429) all failed. Neither trypsinization, boiling or extraction with 85% phenol solution yielded any flavin. This is similar to the result reported in the literature on D-amino acid oxidase from Aspergillus niger, where the authors also were unable to detect any flavin (Kishore, G. and Vaidyanathan, C.S. (1976). India.n J. Biochem. Biophys. 13, 216-222).

The pure enzyme in 20 mM pyrophosphate buffer at pH 8.3 was stable in the frozen state. Thawing and freezing did not destroy the activity. At 8°C, the activity dropped very slowly and the stability at room temperature was relatively low. To improve the thermal stability, the enzyme was immobilized by different methods. In one example, the addition of 17.5 U/ml of enzyme, obtained after Step 3, to 1 ml of CNBr-activated "Sepharose 4B"R at 30°C for 30 min. in 0.1 M borate buffer, pH 8.3, resulted in sustained activity of 7 U/ml "Sepharose".

By replacing the growth medium used by Berg and Rodden (Berg, C.P., and Rodden, F.A. (1976). Anal. Biochem. 71,214-222), the cultivation time could be reduced about 5 times in the present invention. The addition of 0.2% DL-methionine to the medium gave a fivefold increase of the enzyme yield.

## Claims

1. Trigonopsis variabilis D-amino acid oxidase in pure form, as seen by electrophoresis, said D-amino acid oxidase having specific activity against cephalosporin C characterized in that said D-amino acid oxidase has an isoelectric point of about 4.6 and exists in its active form as a dimer with a molecular weight of about 86,000 and with two subunits.

2. The D-amino acid oxidase of claim 1, characterized in that it has been isolated from Trigonopsis variabilis.

3. An immobilized form of the D-amino acid oxidase of claim 1.

4. The immobilized D-amino acid oxidase of claim 3, wherein the D-amino acid oxidase is conjugated with cyanogen-bromide-activated Sepharose.

5. A method for isolating the D-amino acid oxidase of claim 1 which comprises :

(a) acidifying and heating a crude cell extract of Trigonopsis variabilis to obtain a precipitate and supernatant fraction ;

(b) treating said supernatant fraction obtained in step (a) with sufficient ammonium sulfate to obtain a second precipitate, said second precipitate containing the D-amino acid oxidase of claim 1 ; and

(c) resuspending the precipitate obtained in step (b) and collecting the D-amino acid oxidase by isoelectric

precipitation at a pH of about 4.6.

6. The method of claim 5, wherein the crude cell extract is acidified by adding acetic acid.

7. The method of claim 6, wherein the crude cell extract is acidified to a pH of about 4 to 6.

8. The method of claim 7, wherein the pH is about 5.1 to 5.3.

9. The method of claim 8, wherein the pH is about 5.3.

10. The method of claim 5, which further comprises the step of removing any precipitate formed after acidification prior to heating.

11. The method of claim 5, which further comprises the step of adding D,L-methionine to the crude cell extract prior to heating.

12. The methof of claim 11, wherein the D,L-methionine is added in sufficient quantity to obtain a final concentration of about 25 mM.

13. The method of claim 5, wherein said crude extract is heated to a temperature of about 40 to about 60°C.

14. The method of claim 13, wherein said temperature is about 40 to about 50°C.

15. The method of claim 14, wherein said temperature is about 50°C.

16. The method of claim 5, which further comprises the step of dialyzing the supernatant fraction of step (a) prior to treating with ammonium sulfate.

17. The method of claim 16, wherein said dialysis is against a buffer comprising 20 mM sodium pyrophosphate, pH 8.3.

18. The method of claim 16, which further comprises the step of concentrating said supernatant fraction after dialysis prior to treating with ammonium sulfate.

19. The method of claim 18, wherein said concentration is accomplished by evaporation.

20. The method of claim 5, wherein said treatment with ammonium sulfate comprises :

(a) adding ammonium sulfate to said supernatant fraction to obtain an ammonium sulfate concentration of about 30% by weight and removing the resulting precipitate ; and

(b) adding additional ammonium sulfate to the supernatant of (a) to obtain an ammonium sulfate concentration of about 55% and collecting the resulting precipitate.

21. The method of claim 5, which further comprises the step of dialyzing the D-amino acid oxidase-containing second precipitate prior to collecting by isoelectric precipitation.

22. The method of claim 21, wherein said dialysis is against a buffer comprising 20 mM sodium pyrophosphate, pH 8.3.

23. The method of claim 5, wherein the isoelectric precipitation comprises :

(a) dialyzing said second precipitate against a buffer that comprises 25 mM sodium acetate, pH 5.1, and removing any precipitate that remains after said dialyzing step ; and

(b) dialyzing the solution obtained from said dialyzing step against a buffer that comprises 100 mM sodium acetate, pH 4.6, and collecting the purified D-amino acid oxidase precipitate.

24. The method of claim 5, wherein further comprises the step of purifying the D-amino acid oxidase obtained by the isoelectric precipitation by gel electrophoresis.

25. A method for isolating the D-amino acid oxidase of claim 1 from Trigonopsis variabilis, characterized in that

(a) cells of Trigonopsis variabilis are disintegrated for forming a crude extract which is acidified ;

(b) the acidified crude extract is heated to 50°C, and the precipitate formed is removed and the supernatant dialyzed ;

(c) the product from (b) is precipitated with ammonium sulfate and dialyzed ; and

(d) D-amino acid oxidase is isolated by isoelectric precipitation.

26. A method for isolating the D-amino acid oxidase of claim 1 from Trignopsis variabilis which comprises:

(a) acidifying a crude cell extract of Trigonopsis variabilis by adding acetic acid until the pH of the extract is about 5.3 to form a precipitate and supernatant fraction ;

(b) adding D,L-methionine to said supernatant to a final concentration of 25 mM ;

(c) heating said supernatant to 50°C and maintaining it at 50°C for ten minutes to form a second precipitate and supernatant fraction ;

(d) dialyzing said supernatant obtained in step (c) against 20 mM sodium pyrophosphate buffer, pH 8.3 ;

(e) adding sodium ammonium sulfate to the solution prepared in step (d) to a final concentration of 30% and removing the resulting precipitate ;

(f) adding solid ammonium sulfate to the solution prepared in step (e) to a final concentration of 55% and collecting the resulting precipitate ;

(g) dialyzing said precipitate prepared in step (f) first against 20 mM sodium pyrophosphate buffer, pH 8.3, then against 25 mM sodium acetate buffer, pH 5.1, and removing any undissolved precipitate that remains after dialysis ; and

(h) dialyzing the solution obtained in step (g) against 100 mM sodium acetate buffer, pH 4.6, and collecting the-D-amino acid oxidase-containing precipitate.

## Patentansprüche

1. Trigonopsis variabilis D-Aminosäureoxydase in, gemäss elektrophoretischer Analyse, reiner Form, wobei die D-Aminosäureoxydase spezifische Aktivität gegenüber Cephalosporin C aufweist, dadurch **gekennzeichnet**, dass die D-Aminosäureoxydase einen isoelektrischen Punkt von etwa 4,6 hat und in ihrer aktiven Form als ein Dimer mit einem Molgewicht von etwa 86 000 und mit zwei Untereinheiten vorliegt.

2. D-Aminosäureoxydase nach Anspruch 1, dadurch **gekennzeichnet**, dass sie von Trigonopsis variabilis isoliert worden ist.

3. Immobilisierte Form der D-Aminosäureoxydase nach Anspruch 1.

4. Immobilisierte D-Aminosäureoxydase nach Anspruch 3, dadurch **gekennzeichnet**, dass die D-Aminosäureoxydase mit cyanbromidaktivierter Sepharose konjugiert ist.

5. Verfahren zur Isolierung der D-Aminosäureoxydase nach Anspruch 1, umfassend

(a) das Ansäuern und das Erwärmen eines rohen Zellenextrakts von Trigonopsis variabilis, um einen Niederschlag und eine obenstehende Fraktion zu erhalten ;

(b) die Behandlung der in Stufe (a) erhaltenen, obenstehenden Fraktion mit genügend Ammoniumsulfat, um einen zweiten Niederschlag zu erhalten, der die D-Aminosäureoxydase nach Anspruch 1 enthält ; und

(c) das Resuspendieren des in Stufe (b) erhaltenen Niederschlags und das Auffangen der D-Aminosäureoxydase durch isoelektrische Ausscheidung bei einem pH von etwa 4,6.

6. Verfahren nach Anspruch 5, dadurch **gekennzeichnet**, dass der rohe Zellenextrakt durch Zugabe von Essigsäure angesäuert wird.

7. Verfahren nach Anspruch 6, dadurch **gekennzeichnet**, dass der rohe Zellenextrakt auf ein pH von etwa 4 bis 6 angesäuert wird.

8. Verfahren nach Anspruch 7, dadurch **gekennzeichnet**, dass das pH etwa 5,1 bis 5,3 beträgt.

9. Verfahren nach Anspruch 8, dadurch **gekennzeichnet**, dass das pH etwa 5,3 beträgt.

10. Verfahren nach Anspruch 5, das ausserdem die Beseitigung jedes nach der Ansäuerung vor der Erwarmung gebildeten Niederschlags umfasst.

11. Verfahren nach Anspruch 5, das ausserdem die Zugabe von D,L-methionin zum rohen Zellenextrakt vor der Erwärmung umfasst.

12. Verfahren nach Anspruch 11, dadurch **gekennzeichnet**, dass das D,L-methionin in genügender Menge zugegeben wird, um eine endgültige Konzentration von etwa 25 mM zu erhalten.

13. Verfahren nach Anspruch 5, dadurch **gekennzeichnet**, dass der rohe Zellenextrakt auf eine Temperatur von etwa 40 bis etwa 60°C erwärmt wird.

14. Verfahren nach Anspruch 13, dadurch **gekennzeichnet**, dass die Temperatur etwa 40 bis etwa 50°C beträgt.

15. Verfahren nach Anspruch 14, dadurch **gekennzeichnet**, dass die Temperatur etwa 50°C beträgt.

16. Verfahren nach Anspruch 5, das ausserdem die Dialyse der obenstehenden Fraktion nach Stufe (a) vor der Behandlung mit Ammoniumsulfat umfasst.

17. Verfahren nach Anspruch 16, dadurch **gekennzeichnet**, dass die Dialyse gegen einen Puffer umfassend 20 mM Natriumpyrophosphat, pH 8,3, erfolgt.

18. Verfahren nach Anspruch 16, das ausserdem das Konzentrieren der obenstehenden Fraktion nach der Dialyse vor der Behandlung mit Ammoniumsulfat umfasst.

19. Verfahren nach Anspruch 18, dadurch **gekennzeichnet**, dass das Konzentrieren durch Verdunstung durchgeführt wird.

20. Verfahren nach Anspruch 5, dadurch **gekennzeichnet**, dass die Behandlung mit Ammoniumsulfat folgendes umfasst

(a) die Zugabe von Ammoniumsulfat zu der obenstehenden Fraktion, um eine Ammoniumsulfat-Konzentration von etwa 30 Gew.-% zu erhalten, und die Beseitigung des sich dadurch ergebenden Niederschlags; und

(b) die Zugabe von zusätzlichem Ammoniumsulfat zu der obenstehenden Fraktion nach (a), um eine Ammoniumsulfat-Konzentration von etwa 55% zu erhalten, und das Auffangen des sich dadurch ergebenden Niederschlags.

21. Verfahren nach Anspruch 5, das ausserdem die Dialyse des D-Aminosäureoxydase enthaltenden zweiten Niederschlags vor dem Auffangen durch isoelektrische Ausscheidung umfasst.

22. Verfahren nach Anspruch 21, dadurch **gekennzeichnet**, dass die Dialyse gegen einen Puffer umfassend 20 mM Natriumpyrophosphat, pH 8,3, erfolgt.

23. Verfahren nach Anspruch 5, dadurch **gekennzeichnet,** dass die isoelektrische Ausscheidung folgendes umfasst

(a) die Dialyse des zweiten Niederschlags gegen einen Puffer, der 25 mM Natriumacetat, pH 5,1, enthält, und die Beseitigung jedes nach der Dialyse verbleibenden Niederschlags ; und

(b) die Dialyse der von der genannten Dialyse erhaltenen Lösung gegen einen Puffer, der 100 mM Natriumacetat, pH 4,6, enthält, und das Auffangen des gereinigten Niederschlags der D-Aminosäureoxydase.

24. Verfahren nach Anspruch 5, das ausserdem die Reinigung der durch die isoelektrische Ausscheidung erhaltenen D-Aminosäureoxydase durch Gelelektrophorese umfasst.

25. Verfahren zur Isolierung der D-Aminosäureoxydase nach Anspruch 1 von Trigonopsis variabilis, dadurch **gekennzeichnet**, dass

(a) Zellen von Trigonopsis variabilis zerlegt werden, um einen Rohextrakt zu bilden, der angesäuert wird;

(b) der angesäuerte Rohextrakt auf 50°C erwärmt, der gebildete Niederschlag beseitigt, und die obenstehende Fraktion dialysiert wird ;

(c) das Produkt aus (b) mit Ammoniumsulfat abgesetzt und dialysiert wird ; und

(d) D-Aminosäureoxydase durch isoelektrische Ausscheidung isoliert wird.

26. Verfahren zur Isolierung der D-Aminosäureoxydase nach Anspruch 1 von Trigonopsis variabilis, umfassend

(a) das Ansäuern eines rohen Zellenextrakts von Trigonopsis variabilis durch Zugabe von Essigsäure, bis das pH des Extrakts etwa 5,3 beträgt, um einen Niederschlag und eine obenstehende Fraktion zu erhalten;

(b) die Zugabe von D, L-methionin zu der obenstehenden Fraktion bis zu einer endgültigen Konzentration von 25 mM ;

(c) das Erwärmen der obenstehenden Fraktion auf 50°C und das Erhalten derselben bei 50°C zehn Minuten lang, um einen zweiten Niederschlag und eine obenstehende Fraktion zu erhalten ;

(d) die Dialyse der in Stufe (c) erhaltenen, obenstehenden Fraktion gegen einen Puffer, der 20 mM Natriumpyrophosphat, pH 8,3, enthält ;

(e) die Zugabe von Natriumammoniumsulfat zu der in Stufe (d) gebildeten Lösung bis zu einer endgültigen Konzentration von 30%, und die Beseitigung des sich dadurch ergebenden Niederschlags ;

(f) die Zugabe von festem Ammoniumsulfat zu der in Stufe (e) gebildeten Lösung bis zu einer endgültigen Konzentration von 55%, und das Auffangen des sich dadurch ergebenden Niederschlags ;

(g) die Dialyse des in Stufe (f) gebildeten Niederschlags erst gegen einen Puffer, der 20 mM Natriumpyrophosphat, pH 8,3, enthält, danach gegen einen Puffer, der 25 mM Natriumacetat, pH 5,1, enthält, und die Beseitigung jedes nach der Dialyse verbleibenden, unaufgelösten Niederschlags ; und

(h) die Dialyse der in Stufe (g) erhaltenen Lösung gegen einen Puffer, der 100 mM Natriumacetat, pH 4,6, enthält, und das Auffangen des D-Aminosäureoxydase enthaltenden Niederschlags.

## Revendications

1. La D-aminoacide-oxydase de Trigonopsis variabilis sous forme pure, d'après l'électrophorèse, ladite D-aminoacide-oxydase ayant une activité spécifique contre la céphalosporine C, caractérisée en ce que ladite D-aminoacide-oxydase a un point isoélectrique d'environ 4,6 et existe sous sa forme active sous forme d'un dimère d'un poids moléculaire d'environ 86 000 et ayant 2 sous-unités.

2. La D-aminoacide-oxydase selon la revendication 1, caractérisée en ce qu'elle a été isolée à partir de Trigonopsis variabilis.

3. Une forme immobilisée de la D-aminoacide-oxydase selon la revendication 1.

4. La D-aminoacide-oxydase immobilisée selon la revendication 3, dans laquelle la D-aminoacide-oxydase est conjuguée avec la Sépharose activée par le bromure de cyanogène.

5. Un procédé pour isoler la D-aminoacide-oxydase selon la revendication 1, qui comprend les étapes suivantes :

(a) on acidifie et on chauffe un extrait cellulaire brut de Trigonopsis variabilis pour obtenir un précipité et une fraction surnageante ;

(b) on traite ladite fraction surnageante obtenue dans l'étape (a) avec suffisamment de sulfate d'ammonium pour obtenir un second précipité, ledit second précipité contenant la D-aminoacide-oxydase selon la revendication 1 ; et

(c) on remet en suspension le précipité obtenu dans l'étape (b) et on recueille la D-aminoacide-oxydase par précipitation isoélectrique à un pH d'environ 4,6.

6. Le procédé selon la revendication 5, dans lequel l'extrait cellulaire brut est acidifié par addition d'acide

acétique.

7. Le procédé selon la revendication 6, dans lequel l'extrait cellulaire brut est acidifié à un pH d'environ 4 à 6.

8. Le procédé selon la revendication 7, dans lequel le pH est d'environ 5,1 à 5,3.

9. Le procédé selon la revendication 8, dans lequel le pH est d'environ 5,3.

10. Le procédé selon la revendication 5, qui comprend en outre avant le chauffage l'étape de séparation d'un éventuel précipité formé après acidification.

11. Le procédé selon la revendication 5, qui comprend en outre avant le chauffage l'étape d'addition de D,L-méthionine à l'extrait cellulaire brut.

12. Le procédé selon la revendication 11, dans lequel on ajoute la D,L-méthionine en quantité suffisante pour obtenir une concentration finale d'environ 25 mM.

13. Le procédé selon la revendication 5, dans lequel ledit extrait brut est chauffé à une température d'environ 40 à environ 60°C.

14. Le procédé selon la revendication 13, dans lequel ladite température est d'environ 40 à environ 50°C.

15. Le procédé selon la revendication 14, dans lequel ladite température est d'environ 50°C.

16. Le procédé selon la revendication 5, qui comprend en outre l'étape de dialyse de la fraction surnageante de l'étape (a) avant le traitement par le sulfate d'ammonium.

17. Le procédé selon la revendication 16, dans lequel ladite dialyse est contre un tampon comprenant du pyrophosphate de sodium 20 mM, à pH 8,3.

18. Le procédé selon la revendication 16, qui comprend en outre l'étape de concentration de ladite fraction surnageante après dialyse avant le traitement par le sulfate d'ammonium.

19. Le procédé selon la revendication 18, dans lequel ladite concentration est effectuée par évaporation.

20. Le procédé selon la revendication 5, dans lequel ledit traitement par le sulfate d'ammonium comprend les étapes suivantes :

(a) on ajoute du sulfate d'ammonium à ladite fraction surnageante pour obtenir une concentration en sulfate d'ammonium d'environ 30% en poids et on sépare le précipité résultant ; et

(b) on ajoute encore du sulfate d'ammonium à la fraction surnageante de l'étape (a) pour obtenir une concentration en sulfate d'ammonium d'environ 55% et on recueille le précipité résultant.

21. Le procédé selon la revendication 5, qui comprend en outre l'étape de dialyse du second précipité contenant la D-aminoacide-oxydase avant de la recueillir par précipitation isoélectrique.

22. Le procédé selon la revendication 21, dans lequel ladite dialyse est contre un tampon contenant du pyrophosphate de sodium 20 mM, à pH 8,3.

23. Le procédé selon la revendication 5, dans lequel la précipitation isoélectrique comprend les étapes suivantes :

(a) on dialyse ledit second précipité contre un tampon qui comprend de l'acétate de sodium 25 mM, à pH 5,1, et on sépare le précipité éventuel qui reste après ladite étape de dialyse ; et

(b) on dialyse la solution obtenue dans ladite étape de dialyse contre un tampon qui comprend de l'acétate de sodium 100 mM a pH 4,6 et on recueille le précipité de D-aminoacide-oxydase purifiée.

24. Le procédé selon la revendication 5, qui comprend en outre l'étape de purification par électrophorèse sur gel de la D-aminoacide-oxydase obtenue par la précipitation isoélectrique.

25. Un procédé pour isoler la D-aminoacide-oxydase selon la revendication 1 de Trigonopsis variabilis, caractérisé en ce que :

(a) les cellules de Trigonopsis variabilis sont désintégrées pour former un extrait brut qui est acidifié ;

(b) l'extrait brut acidifié est chauffé à 50°C et le précipité formé est séparé et la fraction surnageante est dialysée ;

(c) le produit de l'étape (b) est précipité par le sulfate d'ammonium et dialysé ; et

(d) la D-aminoacide-oxydase est isolée par précipitation isoélectrique.

26. Un procédé pour isoler la D-aminoacide-oxydase selon la revendication 1 de Trigonopsis variabilis qui comprend les étapes suivantes :

(a) on acidifie un extrait cellulaire brut de Trigonopsis variabilis en ajoutant de l'acide acétique jusqu'à ce que le pH de l'extrait soit d'environ 5,3 pour former un précipité et une fraction surnageante ;

(b) on ajoute de la D,L-méthionine à ladite fraction surnageante jusqu'à une concentration finale de 25 mM;

(c) on chauffe ladite fraction surnageante à 50°C et on la maintient à 50°C pendant 10 min pour former un second précipité et une fraction surnageante ;

(d) on dialyse ladite fraction surnageante obtenue dans l'étape (c) contre un tampon au pyrophosphate de sodium 20 mM à pH 8,3 ;

(e) on ajoute du sulfate d'ammonium sodium à la solution préparée dans l'étape (d) jusqu'à une concentration finale de 30% et on sépare le précipité résultant ;

(f) on ajoute du sulfate d'ammonium solide à la solution préparée dans l'étape (e) jusqu'à une concentration finale de 55% et on recueille le précipité résultant ;

(g) on dialyse ledit précipité préparé dans l'étape (f) d'abord contre un tampon au pyrophosphate de sodium 20 mM à pH 8,3, puis contre un tampon à l'acétate de sodium 25 mM à pH 5,1 et on sépare le précipité non dissous éventuel qui reste après la dialyse ; et

(h) on dialyse la solution obtenue dans l'étape (g) contre un tampon à l'acétate de sodium 100 mM à pH 4,6 et on recueille le précipité contenant la D-aminoacide-oxydase.

Fig.1

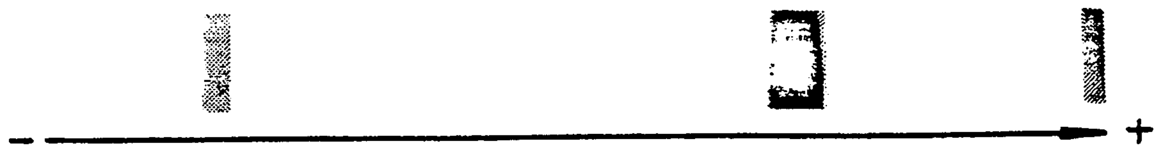


Fig. 2

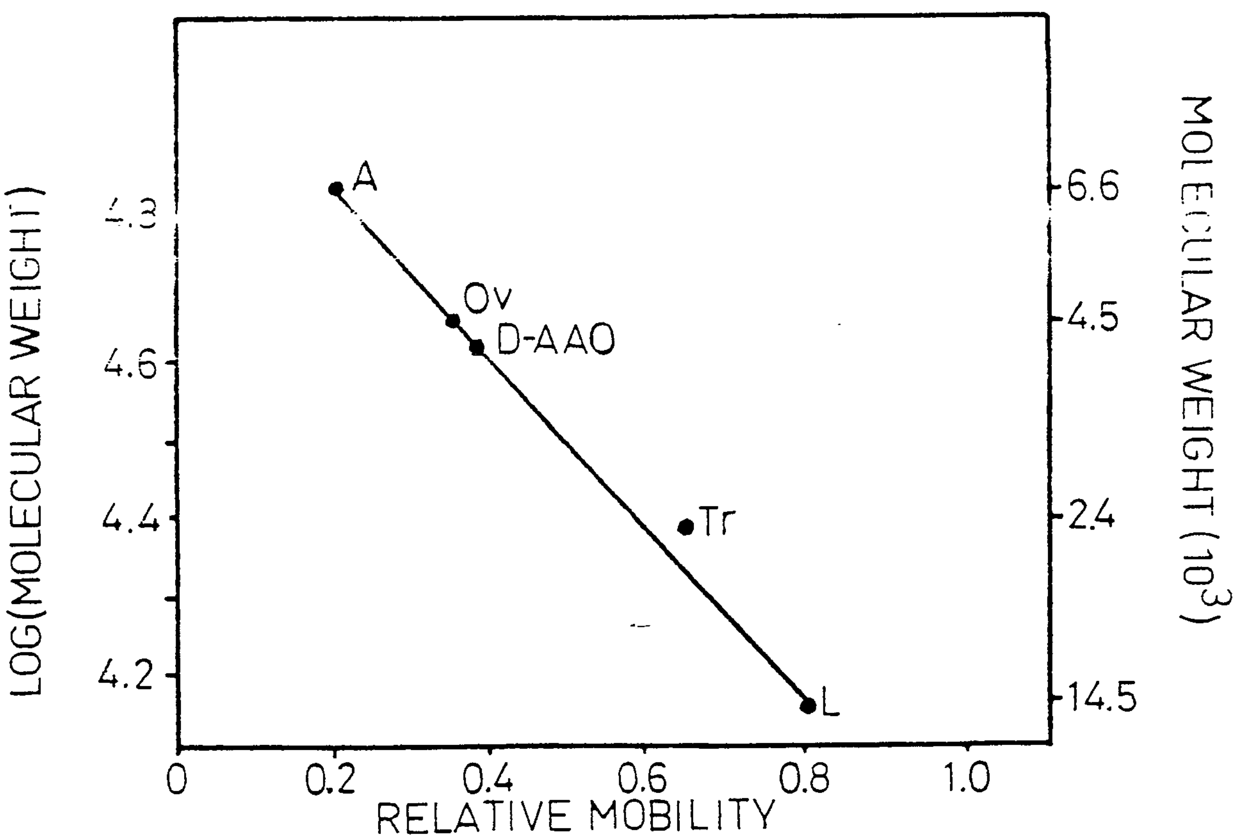


Fig. 3